# EUROPÄISCHE PATENTANMELDUNG

(11) **EP 2 218 478 A1**
(43) Veröffentlichungstag der Anmeldung: **18.08.2010**
(21) Anmeldenummer: 09153032.9
(22) Anmeldetag: 17.02.2009
(51) Int. Cl.: A61N 1/39, G08B 25/00, A61N 1/372, A61B 5/046

(54) **Verfahren und System zur Alarmierung von Rettungskräften bei Herzinfarktpatienten**

(71) Anmelder: Herzsicher AG, 3800 Matten b. Interlaken (CH)
(72) Erfinder: Grossniklaus, Erich, 3503 Gysenstein (CH)
(74) Vertreter: BOVARD AG

(57) **Zusammenfassung**

Es wird ein Verfahren beschrieben und beansprucht, um herzinfarktgefährdete Personen bedeutend schneller defibrillieren zu können. Das Verfahren gestattet es, den Patienten, dem ein Infarkt droht, sehr schnell mit einem Defibrillator und einem Retter, der mit der Bedienung des Gerätes vertraut ist, zusammenzubringen.

Das Verfahren weist folgende Schritte auf: Überwachung der Herzfunktionen mittels eines am Körper des Patienten zu tragenden Überwachungsgeräts mit Funk- und GPS-Funktion; laufende Auswertung der vom Überwachungsgerät abgegebenen Signale und Prüfung auf Anomalien der Herztätigkeit; Auslösung eines Alarmsignals bei ermittelter Anomalie; Übermittlung des Alarmsignals an eine Alarmzentrale; Suche nach nächstliegenden AED-Geräten, nach dem Standort des Patienten und nach mindestens einem mit der Bedienung von AED-Geräten vertrauten Retter; Übermittlung der erhaltenen und erstellten Daten an den mindestens einen Retter; und Zusammenführung von Patient, AED-Gerät und Retter bzw. Rettern.

## Beschreibung

### Gebiet der Erfindung

Die Erfindung betrifft ein Verfahren zur Alarmierung von Rettungskräften bei Herzinfarktpatienten, bei dem ein Defibrillator (bzw. ein so genannter AED) zwecks Vornahme einer Defibrillation gesucht und mit einem Patienten sowie mindestens einem rettenden Bediener des Defibrillators zusammengebracht wird. Ausserdem bezieht sich die vorliegende Erfindung auch auf ein System zur Ausführung des Verfahrens.

Das erfindungsgemässe Verfahren bezweckt eine Alarmierung von Rettungskräften, die an einem herzinfarkt-gefährdeten Patienten insbesondere eine Defibrillation und/oder eine Herzmassage sowie die anderen Massnahmen zur Reanimation der Patienten ausführen sollen. Der Begriff Rettungskräfte im Sinne dieser Anmeldung bezieht sich einerseits auf speziell ausgebildete Sanitäter oder anderes medizinisches Personal, und andererseits aber auch auf Laienhelfer. Das erfindungsgemässe Verfahren und das erfindungsgemässes System sind in diesem Sinne gleichsam geeignet für beide genannten Gruppen von Rettungskräften.

### Technischer Hintergrund

Die Defibrillation ist eine Behandlungsmethode gegen die lebensbedrohlichen Herzrhythmusstörungen Kammerflimmern und Kammerflattern, bei der durch starke Stromstösse die normale Herzaktivität wieder hergestellt werden soll. Das verwendete Gerät nennt man Defibrillator. Viele heutzutage verwendeten Defibrillatoren gehören zur Gruppe der so genannten automatisierten externen Defibrillatoren oder der AEDs. Die AEDs umfassen einen Prozessor, welcher den Herzrhythmus analysieren kann. Auf Grund der Auswertung des Herzrhythmus kann automatisiert entschieden werden, ob eine Defibrillation notwendig ist. Nur bei einem positiven Ergebnis wird nämlich die Defibrillations-Funktion freigeschaltet und kann durch den Anwender ausgelöst werden.

AEDs sind deshalb besonders, aber nicht ausschliesslich, auch für Laienhelfer geeignet. Je nach Funktionsumfang unterstützen AEDs den Laienhelfer durch optische Anweisungen (wie das Aufleuchten von LEDs oder das Darstellen von Handlungsaufforderungen in einem digitalen Display), akustische Anweisungen (klare sprachliche Aufforderungen des Gerätes zu bestimmten Aktionen), Piktogramme (Darstellung der Position der Klebeelektroden) oder auch durch ein eingebautes Metronom. Auch gibt es Varianten für die halbprofessionelle Anwendung mit Darstellung einer EKG-Ableitung. Der Aufbau und die Funktionalität der Defibrillator-Geräte im Allgemeinen und AEDs im Besonderen werden an dieser Stelle als für einen Fachmann wohl bekannt vorausgesetzt.

Die Defibrillation kann ausschliesslich im Rahmen der Reanimation beim so genannten Kammerflimmern, beim Kammerflattern, sowie bei der pulslosen ventrikulären Tachykardie eingesetzt werden.

Kammerflimmern bedeutet, dass durch eine fehlerhafte Erregungsbildung am Herzen alle Herzmuskelzellen zittern, aber zu keiner koordinierten Aktion fähig sind. Dadurch kann das Herz nicht mehr pumpen, es entsteht ein Kreislaufstillstand. Kammerflattern zeigt den gleichen pathologischen Mechanismus wie das Flimmern, unterscheidet sich jedoch in der Frequenz der Flimmerwellen, wobei unterschiedliche Werte für diese Frequenzen angegeben werden (etwa 280/min bis 350/min).

Die ventrikuläre Tachykardie (VT) kann in eine pulslose und eine pulsatile unterschieden werden, wobei die pulslose VT eine absolute Indikation zur Defibrillation darstellt. Die pulsatile VT kann mittels Kardioversion (Sonderform der Defibrillation) beendet werden, sofern eine medikamentöse Therapie wirkungslos bleibt. Die VT zeigt gleichmässige schnelle Erregungen, die von den Herzkammern ausgehen, und nicht wie üblich von den Erregungszentren im Herzvorhof.

Damit das Herz das Blut in die Arterien pumpen kann, muss es sich zusammenziehen. In den beschriebenen Situationen kann sich ein schnell erregtes Herz nicht mehr vollständig oder gar nicht zusammenziehen. Dadurch kann auch kein Blut mehr an die Organe weitergepresst werden, damit diese versorgt werden können. Die Rhythmusstörungen enden daher unbehandelt innerhalb von Minuten tödlich. Kammerflimmern ist die häufigste tödliche Herzrhythmusstörung bei Erwachsenen.

Ursache für die oben beschriebenen Herzrhythmusstörungen kann zum Beispiel ein Herzinfarkt sein, aber auch Stromschläge oder Intoxikationen kommen als mögliche Herzrhythmusstörungserreger in Frage.

Die Ausführung der Defibrillation im Einzelnen soll hier nicht näher beschrieben werden, da diese Behandlungsmethode nicht Gegenstand der vorliegenden Erfindung ist und von einem Fachmann wohl verstanden wird. Dazu wird auf die Fachliteratur und auch auf entsprechende grundlegende Artikel in Lexika (z.B. im Internet-Lexikon Wikipedia, http://de.wikipedia.org) verwiesen. Das Entscheidende bei der Anwendung der Defibrillation - natürlich wenn sie medizinisch indiziert ist - ist die schnelle Anwendung. Je schneller defibrilliert wird, desto höher sind die Überlebenschancen des Patienten. Es wurde gefunden, dass jede Minute ohne Anwendung der Defibrillation die Wahrscheinlichkeit einer erfolgreichen Wiederbelebung des Patienten um ca. 10% verringert.

Die Wartezeiten bis zur Anwendung einer Defibrillation hängen einerseits von der Verfügbarkeit einerseits eines Defibrillators und andererseits von der Verfügbarkeit und Erreichbarkeit des Bedienungspersonals ab, welches ein solches Gerät bedienen kann. Dabei ist man natürlich bestrebt, diese Wartezeiten abzukürzen. Aus diesem Grunde sind in den letzten Jahren mehr und mehr öffentliche Defibrillatoren zur Verfügung gestellt worden, denn wenn ein solches Gerät schnell zugänglich ist, besteht keine Notwendigkeit mehr, einen Krankenwagen herbeizurufen und den Patienten in ein Krankenhaus zu bringen, wo erst die Defibrillation stattfinden kann. Dabei handelt es sich sowohl um ortsfeste Geräte, die nach Art einer Notrufsäule zu bedienen sind, wie sie beispielsweise vom Strassenverkehr her bekannt ist, und andererseits auch um mobile Geräte, die in Kranken- oder andere Wagen eingebaut sind, welche wiederum in den Spitälern, Rettungsstationen oder anderen Standorten bereit stehen.

Allerdings ist neben der Zurverfügungstellung der Defibrillatoren auch essentiell, dass ihre Position leicht nachvollziehbar ist. Ein Defibrillator, welcher zwar vorhanden, jedoch nicht oder nur schwer auffindbar ist, erfüllt seine Aufgabe auch nicht. Aus diesem Grund kann es vorkommen, dass ein Patient vor der Reanimation auf einen Krankenwagen warten muss, obwohl ganz in der Nähe ein Defibrillator einsatzbereit wäre.

Andererseits konnte auch die Aufgabe, geeignet ausgebildete Personen bei einem Notfall, bei dem eine Defibrillation erforderlich ist, an die Stelle des Notfalls zu bringen, bisher nicht zufriedenstellend gelöst werden. Von der (in der Schweiz meist telefonisch an den Notruf 144 bzw. international 112 erfolgten) Meldung des Notfalls an eine dafür vorgesehene Station, in der weitere Massnahmen eingeleitet werden, bis zur Defibrillation vergeht eine zu lange Zeitspanne. Obwohl eine sichere und effiziente Bedienung eines AEDs wie oben beschrieben auch für Laien möglich wäre, ist der Umgang mit diesen Geräten für viele Menschen immer noch nicht geläufig. Deshalb ist es nämlich in der Regel davon auszugehen, dass die Bedienung des Defibrillators für viele der sich in der Umgebung des Patienten befindenden Personen nicht möglich ist, da eben nur sehr wenige Personen mit einem solchen Gerät wirklich zurecht kommen. Wenn Personen, die nicht geübt sind, erst die Bedienungsanleitung lesen müssen, bevor sie eine Reanimation einleiten können, besteht selbstverständlich die Gefahr, dass für den Patienten jede geleistete Hilfe zu spät kommen wird.

### Erfindungsbeschreibung

Die Erfindung hat sich zur Aufgabe gestellt, dieses Problem zu lösen und ein Verfahren sowie ein zur Durchführung des Verfahrens geeignetes System zu entwickeln, das eine schnelle Versorgung eines herzinfarkt-bedrohten Patienten, der durch Defibrillation und/oder weitere Reanimationsmassnahmen zu behandeln ist, ermöglicht.

Das erfindungsgemässe Verfahren ist im kennzeichnenden Teil des ersten unabhängigen Patentanspruches definiert, während das zur Verfahrensdurchführung geeignete System der Erfindung den Gegenstand des zweiten unabhängigen Patentanspruches bildet. Besondere Ausführungsformen sind in den abhängigen Ansprüchen niedergelegt.

Insbesondere wird das erfindungsgemässe Verfahren durch die folgenden Schritte gekennzeichnet:
a) Überwachung der Herzfunktionen mittels eines am Körper des Patienten zu tragenden Überwachungsgeräts mit Funk- und GPS-Funktion;
b) laufende Auswertung der vom Überwachungsgerät abgegebenen Signale und Prüfung auf Anomalien der Herztätigkeit;
c) Auslösung eines Alarmsignals bei ermittelter Anomalie;
d) Übermittlung des Alarmsignals an eine Alarmzentrale;
e) Suche nach nächstliegenden AED-Geräten, nach dem Standort des Patienten und nach mindestens einem mit der Bedienung von AED-Geräten vertrauten Retter;
f) Übermittlung der im Schritt e) erhaltenen und erstellten Daten an den mindestens einen Retter; und
g) Zusammenführung von Patient, AED-Gerät und Retter bzw. Rettern.

Voraussetzung für das erfindungsgemässe Verfahren ist es, dass der infarktgefährdete Patient ein Kontroll- bzw. Überwachungsgerät am Körper trägt. Ähnliche Geräte sind bekannt und werden normalerweise einem Herzpatienten vom Kardiologen zwecks Tagesanalyse für 24 Stunden zum Tragen verordnet. Das Gerät zeichnet üblicherweise die Zeit, die Pulsfrequenz und allenfalls den in Intervallen gemessenen Blutdruck auf. Für den hier interessierenden Zweck misst das Gerät kontinuierlich die Herzfrequenz und/oder die Herzströme und wertet die gemessenen Parameter nach bestimmten Kriterien aus. Ähnliche Geräte sind bekannt und lassen sich wegen der fortgeschrittenen Miniaturisierung bequem am Körper tragen. Zusätzlich verfügt das Gerät noch über eine Ortungsfunktion (zum Beispiel in Anwendung des GPS = Global Positioning System), wodurch die jeweilige Position des Geräts (und damit die Position seines Trägers) auf der Erdoberfläche bekannt ist.

Solche Kontroll- bzw. Überwachungsgeräte können dedizierte Geräte sein, welche nur diesem bestimmten Zweck dienen, aber es können selbstverständlich auch Multizweckgeräte eingesetzt werden, welche für diese Funktionalität aus- bzw. umgerüstet werden. Beispielsweise ist es denkbar, dass ein PDA, Mobiltelefon oder ein Smartphone durch das Anschliessen von Messelektroden und die Installation entsprechender Software zu einem solchen Kontroll- bzw. Überwachungsgerät umgerüstet werden. Selbstverständlich sind auch andere Geräte denkbar.

Beim erfindungsgemässen Verfahren werden die Standorte der einzelnen Defibrillatoren (bzw. der AEDs) benötigt. Diese sind aus diesem Grund in einer Datenbank niedergelegt, welche laufend aktualisiert wird. Die mobilen AED-Geräte sind vorteilhaft auch mit einer GPS-Funktion ausgerüstet, welche es erlaubt, in jedem Augenblick den Standort dieses Gerätes zu kennen. In der Datenbank sind auch die GPS-Daten der mobilen AED-Geräte gespeichert und jederzeit abrufbar, so dass zu jeder Zeit die Daten sämtlicher einsatzfähiger AED-Apparate verfügbar sind.

In einer weiteren Datenbank sind die interessierenden Daten des Patienten gespeichert. Dazu gehören beispielsweise Besonderheiten des Patienten, die bei einem AED-Eingriff eine Rolle spielen können, und insbesondere auch die laufend veränderlichen Positionsdaten des Patienten.

Eine dritte Datenbank enthält schliesslich die Koordinaten und alle anderen wichtigen Daten der eingetragenen Retter, die bei einem Notfall aufgeboten werden können. Im Normalfall sind auch die Retter mit GPS-Geräten ausgerüstet, so dass ihr Standort jederzeit bekannt ist. Alternativ ist es auch denkbar, dass die Retter sich bei einer Standortänderung durch eine andere Positionserkennungsart in der Datenbank anmelden (z.B. durch einen Telefonanruf auf eine bestimmte Nummer und die Eingabe eines Codes, welcher für den Standort steht). Auch eine manuelle Eingabe übers Internet oder durch eine Bedienungsperson ist durchaus denkbar.

Das erfindungsgemässe Verfahren besteht aus mehreren Schritten. Der erste Schritt umfasst die Auslösung eines Alarms. Dies kann automatisch oder manuell geschehen.

Bei einem manuell ausgelösten Alarm drückt der Patient auf einen Knopf, der sich an seinem Überwachungsgerät befindet. Dies kann beispielsweise dann erfolgen, wenn sich der Patient schlecht fühlt und einen Verdacht auf ein Herzproblem hat, oder aus anderen Gründen, etwa zur Ausführung einer Kontrolle oder zur Prophylaxe. Als Folge des Knopfdruckes werden die aktuellen, vom Gerät gespeicherten Daten einschliesslich des letzten EKGs (= Elektrokardiogramm) an eine Alarmzentrale übermittelt, wo sie von einer Maschine oder von einem Arzt ausgewertet werden. Selbstverständlich übermittelt das Gerät auch die jeweiligen GPS-Positionsdaten. Die Auslösung eines Alarms zur Einleitung einer Defibrillation wird dann vom auswertenden Arzt oder einer anderen befugten Person veranlasst.

Bei einem automatischen Alarm läuft das erfindungsgemässe Verfahren etwas anders ab:

Die vom Überwachungsgerät aufgenommenen und gespeicherten Herzsignale werden kontinuierlich ausgewertet und laufend mit den gespeicherten Normalwerten verglichen. Beim Auftreten von Anomalien, beispielsweise einem beginnenden Herzflimmern, wird sofort ein Alarmsignal über Funk an die Alarmzentrale übermittelt. An dieser Stelle möchten wir betonen, dass unter Funk selbstverständlich sämtliche drahtlosen Datenübermittlungsverfahren verstanden werden, welche nicht nur den Funk im klassischen Sinne umfassen müssen. Dieses Alarmsignal enthält bereits die GPS-Positionsdaten des Patienten. Dies bedeutet, dass ein Alarm bereits ausgelöst wird, bevor sich der Patient der drohenden Gefahr eines Herzunfalls überhaupt definitiv bewusst wird und der Unfall tatsächlich eintritt. Diese rechtzeitige Alarmierung, die innerhalb weniger Sekunden vor sich geht, erhöht die Erfolgsaussichten einer kommenden Behandlung durch Defibrillation beträchtlich.

Auf Grund der Position des Patienten bestimmt die Alarmzentrale (in der Regel automatisch, um keine Zeit zu verlieren) die geographisch am nächsten befindlichen AED-Apparate und ebenfalls die am nächsten befindlichen Retter. Beim erfindungsgemässen Verfahren kann gewählt werden wie viele AED-Geräte und Retter aktiviert werden. Beispielsweise werden zwei AEDs, ein ortsfestes und ein mobiles, ausgewählt, sowie zwei Retter aufgeboten, die beide motorisiert sind, denn es wird nötig sein, den Patienten zum nächsten ortsfesten AED zu transportieren, wenn das mobile AED nicht rechtzeitig eintreffen kann. Wenn mehrere mobile AED-Geräte verfügbar sind, welche sich etwa gleich weit vom Patienten befinden, werden vorzugsweise zwei oder mehrere solcher Geräte aktiviert, wobei dasjenige mobile Gerät den Vorzug hat, das sich am nächsten zum nächsten Retter befindet.

Nachdem die am nächsten beim Patienten liegenden AEDs und Retter geortet sind, werden die Retter alarmiert. Sie erhalten die folgenden Informationen: Position des Patienten, Position des am nächsten liegenden AEDs sowie die Navigationshilfe, damit sie zuerst das AED-Gerät und anschliessend den Patienten finden können. Dafür können besondere Geräte verwendet werden, welche die erforderlichen Schnittstellen aufweisen, so dass eine einfache Alarmierung möglich ist. Es können aber auch herkömmliche Geräte verwendet werden, beispielsweise Mobiltelefone, oder auch die sogenannten Smartphones bzw. PDAs mit der zugehörigen Software.

Parallel zu diesem Alarmierungsverfahren kann die Alarmzentrale, sofern es erforderlich oder sachdienlich ist, auch noch Notrufe über das öffentliche Netz, nämlich den internationalen Notruf 112 (oder 144 für den schweizerischen Rettungsdienst) absetzen. Dabei können neben der Patientenposition selbstverständlich auch noch andere Patienteninformationen (wie die Identifikationsdaten, Alter, allfällige Krankheitsgeschichte, usw.) übermittelt werden.

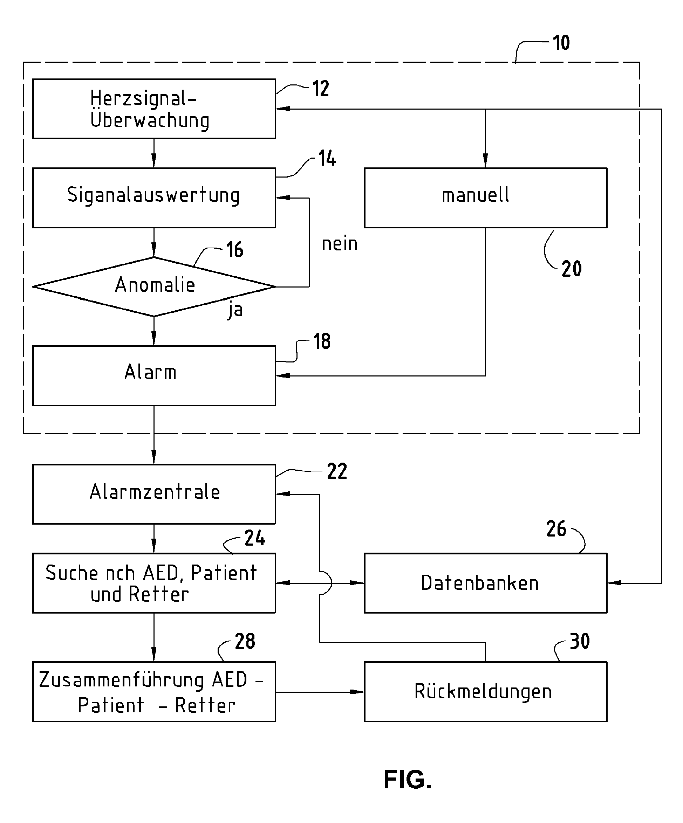

### Kurzbeschreibung der Zeichnung

In der Zeichnung mit einer einzigen Figur ist der Ablauf des erfindungsgemässen Verfahrens an einem Ausführungsbeispiel in Form eines Fliessbildes dargestellt.

### Beschreibung eines Ausführungsbeispiels

In der Figur ist schematisch eine Herzsignal-Überwachung 10 dargestellt; dies geschieht mittels eines Gerätes, das als solches bekannt ist und hier nicht gezeigt wird. Der Patient trägt das (batteriegespeiste) Gerät 10 am Körper.

In kurzen Zeitabständen werden die von der Überwachungsschaltung 12 gesammelten Daten in einer Auswertungsschaltung 14 überprüft und mit gespeicherten Daten verglichen. Wenn im Diskriminator 16 eine Anomalie festgestellt wird, die als beginnendes Herzflimmern oder -flattern ausgelegt wird, löst das Gerät 10 per Funk einen oben beschriebenen Alarm 18 aus. Solange keine Anomalien ermittelt werden, geht der Verfahrensablauf zur Signalauswertung 14 zurück ("Nein-Schleife"). Ein Alarm kann auch manuell mit Hilfe des Elements 20 ausgelöst werden.

Der Alarm gelangt an die Alarmzentrale 22, in welcher zunächst geprüft wird, ob es sich um einen echten Alarm oder nur um eine Anforderung einer Überprüfung der Situation oder des Gerätes 10 handelt. Liegt ein echter Alarm vor, wird im Schritt 24 die Suche nach den AEDs, dem Patienten und den Rettern eingeleitet, wie es oben bereits im Einzelnen beschrieben wurde. Dabei werden die oben beschriebenen Datenbanken 26 herangezogen, denen die erforderlichen Informationen entnommen werden.

Selbstverständlich ist es auch denkbar, das erfindungsgemässe System auch so zu gestalten, dass die Auswertung der vom Überwachungsgerät erfassten Daten nicht auf dem Überwachungsgerät selber, sondern in einer vom Überwachungsgerät getrennten Auswertungseinheit vorgenommen wird. Beispielsweise ist es denkbar, dass die Herzfunktionsdaten über eine Funkverbindung an eine zentrale Auswertungseinheit übermittelt werden, wo sie ausgewertet werden, und wo basierend auf dieser Auswertung nötige Massnahmen getroffen werden. Auf diese Weise ist es insbesondere möglich, Überwachungsgeräte zu bauen, welche einfach gebaut sind und auch sehr wenig Strom verwenden und daher günstig hergestellt und auch ganz praktisch gehandhabt werden können. Auch ist es bei einer zentralen Auswertung von gesammelten Daten möglich, die Aktualisierung der Auswertungssoftware einfach und zentral vorzunehmen. Auch können eventuelle Pannen leichter behoben werden.

Diese zentrale Auswertungseinheit kann beispielsweise als eine gesonderte Einheit innerhalb oder im Rahmen der Alarmzentrale, aber auch als eine vollkommen selbständige Einheit ausgeführt werden, welche mit der Alarmzentrale über einen beliebigen (drahtlosen oder drahtgebundenen) Kommunikationskanal verbunden ist.

Im Schritt 28 findet eine Zusammenführung von Patient, Rettern und AED-Gerät statt, wobei dieser Vorgang entweder an einem ortsfesten AED-Gerät oder, im Falle eines mobilen AEDs, am Standort des Patienten stattfindet. Eine Defibrillation und andere Reanimationsmassnahmen können nun vorgenommen werden. Im Schritt 30 wird eine Rückmeldung an die Alarmzentrale abgegeben, wobei entweder das Überwachungsgerät am Patienten oder ein Rufgerät eines Retters benutzt wird.

Die Erfindung ist nicht auf die beschriebene Ausführungsform beschränkt. Das erfindungsgemässe Verfahren und das zur Ausführung geeignete System können im Rahmen des Beanspruchten verbessert, verändert und/oder ergänzt werden. Beispielsweise kann eine weitere Datenbank im Zusammenwirken mit der Alarmzentrale erstellt werden, in welcher die Ergebnisse der Signalauswertung 14 und/oder sämtliche ausgeführten Verfahren in einer log-Datei gespeichert werden.

Mit der Erfindung wird ein System geschaffen, welches durch äusserst schnelles und wirksames Alarmieren und Aufbieten von Rettungskräften lebensrettend wirkt.

## Patentansprüche

1. Verfahren zur Alarmierung von Rettungskräften für Herzinfarktpatienten, bei dem ein Defibrillator (AED) zwecks Vornahme einer Defibrillation gesucht und mit einem Patienten sowie mindestens einem rettenden Bediener des Defibrillators zusammengebracht wird, **gekennzeichnet durch** die folgenden Schritte:
a) Überwachung der Herzfunktionen mittels eines am Körper des Patienten zu tragenden Überwachungsgeräts mit Funk- und GPS-Funktion;
b) laufende Auswertung der vom Überwachungsgerät abgegebenen Signale und Prüfung auf Anomalien der Herztätigkeit;
c) Auslösung eines Alarmsignals bei ermittelter Anomalie;
d) Übermittlung des Alarmsignals an eine Alarmzentrale;
e) Suche nach nächstliegenden AED-Geräten, nach dem Standort des Patienten und nach mindestens einem mit der Bedienung von AED-Geräten vertrauten Retter;
f) Übermittlung der im Schritt e) erhaltenen und erstellten Daten an den mindestens einen Retter; und
g) Zusammenführung von Patient, AED-Gerät und Retter bzw. Rettern.

2. Verfahren nach Anspruch 1, **dadurch gekennzeichnet, dass** der Schritt b) im Überwachungsgerät selbst erfolgt.

3. Verfahren nach Anspruch 1 oder 2, **dadurch gekennzeichnet, dass** der Alarm in Schritt c) im Überwachungsgerät selbst ausgelöst wird.

4. Verfahren nach Anspruch 1, 2 oder 3, **dadurch gekennzeichnet, dass** der Alarm automatisch ausgelöst und per Funk an die Alarmzentrale übermittelt wird.

5. Verfahren nach Anspruch 1, 2 oder 3, **dadurch gekennzeichnet, dass** der Alarm von Hand ausgelöst und per Funk an die Alarmzentrale übermittelt wird.

6. Verfahren nach einem der vorstehenden Ansprüche, **dadurch gekennzeichnet, dass** die Alarmzentrale in Schritt e) zusätzlich einen Notruf ins öffentliche Telefonnetz absetzt.

7. Verfahren nach einem der vorstehenden Ansprüche, **dadurch gekennzeichnet, dass** die Alarmzentrale in Schritt e) auf Daten zurückgreift, die in mindestens einer Datenbank gespeichert sind, in der sich die Koordinaten der verfügbaren AED-Geräte, der bereitstehenden Rettungskräfte und des Patienten befinden.

8. Verfahren nach einem der vorstehenden Ansprüche, **dadurch gekennzeichnet, das** in Schritt f) eine Rückmeldung an die Alarmzentrale verfasst und weitergeleitet wird, in der ein Erfolg oder Misserfolg der Vorgänge gemeldet wird.

9. System zur Ausführung des Verfahrens nach mindestens einem der vorstehenden Ansprüche, **dadurch gekennzeichnet, dass** es folgende Bestandteile aufweist:
- ein vom Herzpatienten zu tragendes Überwachungsgerät, das mindestens mit Einrichtungen zur Überwachung der Herztätigkeit, zur Auswertung der aus der Überwachung gewonnenen Signale, zur Erzeugung von Alarmsignalen, zum Senden der Signale per Funk und zur Bestimmung der Position mittels GPS ausgestattet ist;
- ein Netz von stationären und/oder mobilen Defibrillatoren (AED-Geräten);
- eine Alarmzentrale, welche mindestens zum Empfang der Alarmsignale vom Überwachungsgerät, zur Suche nach verfügbaren AED-Geräten, nach der Position des Patienten und nach der Position und Verfügbarkeit von Rettern ausgerüstet ist, welche die AED-Geräte bedienen können, und
- mindestens eine Datenbank, welche mindestens die zur Verfahrensdurchführung erforderlichen Daten enthält, darunter die Koordinaten der Position des Patienten, seine medizinischen Daten, die Standorte der AED-Geräte und ihre Betriebsbereitschaft und die örtlichen und persönlichen Koordinaten der Rettungskräfte.

10. System nach Anspruch 9, **dadurch gekennzeichnet, dass** das Überwachungsgerät mit Einrichtungen zur Erzeugung eines Elektrokardiogramms ausgerüstet ist.

11. System nach Anspruch 9 oder 10, **dadurch gekennzeichnet, dass** das Überwachungsgerät mit einer Einrichtung zur Erzeugung eines Alarms von Hand eingerichtet ist, wobei diese Einrichtung so ausgebildet ist, dass dieser manuelle Alarm per Funk an die Alarmzentrale übermittelt wird.

12. System nach Anspruch 9, 10 oder 11, **dadurch gekennzeichnet, dass** in der Alarmzentrale Mittel vorhanden sind, um einen Alarm zusätzlich in das öffentliche Notrufsystem einzugeben.
